# EUROPEAN PATENT APPLICATION

(11) **EP 4 534 150 A1**
(43) Date of publication of application: **09.04.2025**
(21) Application number: 23811767.5
(22) Date of filing: 22.05.2023
(51) Int. Cl.: A61Q 1/02, A61Q 19/00, A61K 8/06, A61K 8/19, A61K 8/25, A61K 8/27, A61K 8/29, A61K 8/34, A61K 8/39, A61K 8/86

(54) **OIL FOR COSMETIC AND COSMETIC CONTAINING SAME**

(30) Priority: 26.05.2022 JP 2022086450; 16.12.2022 JP 2022201487
(71) Applicant: NOF Corporation, Shibuya-ku Tokyo 150-6019 (JP)
(72) Inventor: SHOJI, Ryoka, Kawasaki-shi, Kanagawa 210-0865 (JP); SATO, Megumi, Kawasaki-shi, Kanagawa 210-0865 (JP); SEKIGUCHI, Koji, Kawasaki-shi, Kanagawa 210-0865 (JP)
(74) Representative: Weickmann & Weickmann PartmbB
(86) International application number: PCT/JP2023/018897
(87) International publication number: WO 2023/228895

(57) **Abstract**

The present invention relates to an oil agent for cosmetics, containing 70% by mass to 99% by mass of (A) a primary alcohol having a branched chain and 20 to 30 carbon atoms, and 1% by mass to 30% by mass of (B) a primary alcohol having a branched chain and 12 to 19 carbon atoms, an emulsion cosmetic containing the aforementioned oil agent for cosmetics, (C) a divalent to hexavalent polyhydric alcohol having 3 to 6 carbon atoms, and (D) a non-ionic surfactant having an HLB value of 10 to 18, at a given content ratio, and a cosmetic containing the aforementioned component (A) and component (B), and (F) an inorganic powder having an average particle size of 10 nm to 100 µm, at predefined contents.

According to the present invention, an emulsion cosmetic that is superior in a feeling of thickness when applied to the skin in a high temperature and high humidity state and a feeling of penetration into the skin in a high temperature and high humidity state, and further causes less unpleasant sense of adsorption after application, and a cosmetic that can suppress a feeling of dryness and a sense of friction due to the inclusion of inorganic powder, and can simultaneously achieve a feeling of thickness and a feeling of blending into the skin when applied to the wet skin can be provided.

## Description

### [Technical Field]

The present invention relates to an oil agent for cosmetics and cosmetics containing the agent.

More particularly, the present invention relates to an oil agent for cosmetics that affords a good feeling of thickness when applied to the skin in a high temperature and high humidity state, is superior in a feeling of penetration into the skin in a high temperature and high humidity state, and causes less unpleasant sense of adsorption after application, emulsion cosmetics containing the aforementioned oil agent for cosmetics, and cosmetics containing the aforementioned oil agent for cosmetics and an inorganic powder.

### [Background Art]

Dry skin and rough skin occur not only on the face but also widely on body parts such as hands, legs, and the like, and thus skincare cosmetics have conventionally been used to moisturize the skin and prevent skin dryness. The feeling desired for such skincare cosmetics includes a moisturizing feeling, which is an essential characteristic of skincare cosmetics, as well as a feeling of thickness when applied and a feeling of penetration into the skin. Therefore, there is a demand for an oil agent superior in the feeling of thickness when applied and the feeling of penetration into the skin, as an oil agent to be blended into skincare cosmetics for the purpose of improving the moisturizing feeling.

Patent Literature 1 discloses use of one or more selected from an ester of oleic acid and an alcohol having 1 to 4 carbon atoms, a liquid higher alcohol having 12 to 24 carbon atoms, and a liquid higher fatty acid having 12 to 24 carbon atoms as a skin penetrating component in emulsion-type skin external preparations, for the purpose of improving the feeling of penetration into the skin. However, the technique described in Patent Literature 1 has a problem in that even though an emulsion-type skin external preparation superior in the feeling of penetration into the skin is obtained, the feeling of thickness when applied becomes insufficient as the feeling of penetration into the skin is improved.

Patent Literature 2 proposes an emulsion cosmetic that contains a higher alcohol that is solid at room temperature, a higher alcohol that is liquid at room temperature, phospholipid, and oily component other than the aforementioned higher alcohols at a specific content ratio as the oil component, and that can afford the effect of adjusting the texture of the skin, the effect of smoothing the skin, and the effect of imparting plumpness, resilience, elasticity, and flexibility to the skin the next morning.

In view of convenience, however, skincare cosmetics that can be applied to wet skin after bathing or to the skin in a high temperature and high humidity state after towel drying have been demanded in recent years, and the emulsion cosmetic described in Patent Literature 2 is insufficient in the feeling of penetration into the skin in a high temperature and high humidity state.

Patent Literature 3 discloses an O/W type emulsion skin external composition which contains an oily component that is liquid or semi-solid at 25°C, a multiple-chain surfactant with multiple hydrophilic groups and having multiple long-chain hydrophobic groups and multiple hydrophilic groups in the molecule, and an emulsifying polymer, and which shows superior remaining property of the oily component on the skin and can be used on wet skin.

However, even though the O/W type emulsion skin external composition described in Patent Literature 3 is superior in the feeling of thickness when applied to the wet skin, it is insufficient in the feeling of penetration into the skin.

Furthermore, with the spread of smartphones, navigation systems, and the like in recent times, when applied cosmetics remain on the skin, fingerprints adhere to the liquid crystal display screen and cause problems of unpleasant sense of adsorption such as poor slidability and a sense of being caught on the display screen, from the perspective of operability of liquid crystal display screen. Thus, the development of cosmetics improved in such unpleasant sense of adsorption has been desired.

However, it has been extremely difficult to produce a cosmetic that maintains a feeling of thickness when applied to the skin in a high temperature and high humidity state, is superior in a feeling of penetration into the skin in a high temperature and high humidity state, and has been improved in an unpleasant sense of adsorption.

In addition, cosmetics containing powders, such as foundations and sunscreen cosmetics, use inorganic powders such as white pigments, colored pigments, ultraviolet scattering agents (titanium oxide, zinc oxide, etc.), and the like. These inorganic powders have a coloring effect to make the skin look beautiful and an effect of protecting against ultraviolet rays. On the other hand, in the aforementioned cosmetics, the feeling of use, such as a feeling of thickness when applied, a feeling of blending into the skin, spreadability on the skin, and the like, is considered important in addition to the main functions of coloring and UV protection. When the content of inorganic powder is increased to enhance the above-mentioned functions of coloring and UV protection, a noticeable feeling of dryness and a sense of friction occur when the cosmetic is applied to the skin, and the feeling of use is deteriorated.

For example, Patent Literature 4 proposes a technology for imparting a fresh feeling of use to an oil-in-water emulsion cosmetic containing 2.5% by mass to 30% by mass of an ultraviolet scattering agent having a hydrophobic surface, by using a hydrophobically modified alkyl cellulose as an emulsifier and further using a water-soluble thickener with low salt resistance. However, although the oil-in-water emulsion cosmetic proposed in Patent Literature 4 affords a feeling of use with a good feeling of blending into the skin, there is room for improvement in terms of a feeling of dryness and a sense of friction when applied to the skin.

The above-mentioned Patent Literature 3 discloses an O/W type emulsion skin external composition which contains an oily component that is liquid or semi-solid at 25°C, a multiple-chain surfactant with multiple hydrophilic groups and having multiple long-chain hydrophobic groups and multiple hydrophilic groups in the molecule, and an emulsifying polymer, and which shows superior remaining property of the oily component on the skin. Even though the O/W type emulsion skin external composition described in Patent Literature 3 is superior in the effect of making the oily component remain after use on wet skin and rinsing off, it is insufficient in the feeling of blending into the skin.

Furthermore, cosmetics are used in a wide variety of situations, such as after washing the face, after skin care, after bathing, and so on. When the skin is wet, however, the desired feeling of use is often not obtained. In particular, when a cosmetic is applied directly to wet skin, a problem occurs in that it is difficult to simultaneously achieve a feeling of thickness and a feeling of blending into the skin.

Therefore, a cosmetic has been desired that can suppress a feeling of dryness and a sense of friction due to the inclusion of inorganic powder, and can simultaneously achieve a feeling of thickness and a feeling of blending into the skin when applied to the wet skin.

### [Citation List]

### [Patent Literature]

[Patent Literature 1]
   JP 2007-238488 A
[Patent Literature 2]
   JP 2005-53879 A
[Patent Literature 3]
   JP 2017-178904 A
[Patent Literature 4]
   JP 2015-120682 A

### [Summary of Invention]

### [Technical Problem]

Thus, the present invention aims to provide an oil agent for cosmetics that affords a good feeling of thickness when applied to the skin in a high temperature and high humidity state, is superior in a feeling of penetration into the skin in a high temperature and high humidity state, causes less unpleasant sense of adsorption after application, and is preferred as an oily starting material for cosmetics. In addition, it aims to provide a cosmetic containing the aforementioned oil agent for cosmetics, particularly, an emulsion cosmetic that is superior in a feeling of thickness when applied to the skin in a high temperature and high humidity state and a feeling of penetration into the skin in a high temperature and high humidity state, causes less unpleasant sense of adsorption after application, and is suitable for use on the skin in a wet state and the skin in a high temperature and high humidity state.

Furthermore, the present invention aims to provide a cosmetic that can suppress a feeling of dryness and a sense of friction due to the inclusion of inorganic powder, and can simultaneously achieve a feeling of thickness and a feeling of blending into the skin when applied to the wet skin.

### [Solution to Problem]

The present inventors have conducted intensive studies in view of the above-mentioned matters and found that an oil agent for cosmetics, containing a primary alcohol having a branched chain and 20 to 30 carbon atoms, and a primary alcohol having a branched chain and 12 to 19 carbon atoms each at a specific content can solve the above-mentioned problem. They have conducted further studies and completed the present invention.

In addition, the present inventors have found that the above-mentioned problem in cosmetics containing an inorganic powder can be solved by combining the above-mentioned oil agent for cosmetics and an inorganic powder having an average particle size of 10 nm to 100 µm. They have conducted further studies and completed the present invention.

Accordingly, the present invention provides the following.
[1] An oil agent for cosmetics, comprising 70% by mass to 99% by mass of (A) a primary alcohol having a branched chain and 20 to 30 carbon atoms, and 1% by mass to 30% by mass of (B) a primary alcohol having a branched chain and 12 to 19 carbon atoms.
[2] An emulsion cosmetic comprising 1% by mass to 60% by mass of the oil agent for cosmetics of [1], (C) a divalent to hexavalent polyhydric alcohol having 3 to 6 carbon atoms, and (D) a non-ionic surfactant having an HLB value of 10 to 18, wherein a ratio of the total content of the component (A) and the component (B) to the content of the component (C) [{(A)+(B)}/(C)] is 0.05 to 30 in a mass ratio.
[3] A cosmetic comprising 0.5% by mass to 60% by mass of (A) a primary alcohol having a branched chain and 20 to 30 carbon atoms, 0.01% by mass to 30% by mass of (B) a primary alcohol having a branched chain and 12 to 19 carbon atoms, and 2% by mass to 50% by mass of (F) an inorganic powder having an average particle size of 10 nm to 100 µm, wherein a content ratio of the component (A) to the component (B) [(A)/(B)] is 2.3 to 99 in a mass ratio.

### [Advantageous Effects of Invention]

According to the present invention, an oil agent for cosmetics that has a good feeling of thickness when applied to the skin in a high temperature and high humidity state, is superior in a feeling of penetration into the skin in a high temperature and high humidity state, and causes less unpleasant sense of adsorption after application can be provided.

According to the present invention, moreover, a cosmetic that affords a good feeling of thickness when applied to the skin in a high temperature and high humidity state and a good feeling of penetration into the skin in a high temperature and high humidity state can be provided. In particular, an emulsion cosmetic that is superior in a feeling of thickness when applied to the skin in a high temperature and high humidity state and a feeling of penetration into the skin in a high temperature and high humidity state, causes less unpleasant sense of adsorption after application, is suitable for use on the wet skin after bathing and the skin in a high temperature and high humidity state after towel drying, and is also superior in the operability of a liquid crystal display screen can be provided.

Furthermore, according to the present invention, a cosmetic that can suppress a feeling of dryness and a sense of friction due to the inclusion of inorganic powder, and can simultaneously achieve a feeling of thickness and a feeling of blending into the skin when applied to the wet skin can be provided.

### [Description of Embodiments]

The present invention provides an oil agent for cosmetics.

The oil agent for cosmetics of the present invention contains (A) primary alcohol having a branched chain and 20 to 30 carbon atoms, and (B) primary alcohol having a branched chain and 12 to 19 carbon atoms.

The primary alcohol having a branched chain and 20 to 30 carbon atoms that is used as component (A) in the oil agent for cosmetics of the present invention is not particularly limited as long as it can be used as an oil agent for cosmetics. A primary alcohol having 20 to 28 carbon atoms is preferred, one having 20 to 27 carbon atoms is more preferred, one having 20 to 24 carbon atoms is further preferred, and one having 20 carbon atoms is particularly preferred.

Specific examples of component (A) include 2-octyl-1-dodecanol, 2-decyl-1-dodecanol, 2-decyl-1-tetradecanol, 2-dodecyl-1-hexadecanol, 2-dodecyl-1-heptadecanol, 2-tetradecyl-1-hexadecanol, and the like. Among these, 2-octyl-1-dodecanol, 2-decyl-1-dodecanol, 2-decyl-1-tetradecanol, and 2-dodecyl-1-hexadecanol are preferred, 2-octyl-1-dodecanol, 2-decyl-1-dodecanol, and 2-decyl-1-tetradecanol are more preferred, and 2-octyl-1-dodecanol is particularly preferred.

In the present invention, only one kind of the above-mentioned component (A) may be used or two or more kinds thereof may be used in combination.

As the above-mentioned component (A), commercially available products provided by various companies for use in cosmetics can be used.

The content of component (A) in the oil agent for cosmetics of the present invention is 70% by mass to 99% by mass.

When the content of component (A) exceeds 99% by mass, the feeling of penetration into the skin and the sense of adsorption after application may become poor. From the aspects of the feeling of penetration into the skin and the sense of adsorption after application, the content of component (A) is preferably 97% by mass or less, more preferably 95% by mass or less.

When the content of component (A) is less than 70% by mass, a feeling of thickness when applied to the skin may become insufficient. From the aspect of a feeling of thickness when applied to the skin, the content of component (A) is preferably 75% by mass or more, more preferably 78% by mass or more, further preferably 80% by mass or more.

The primary alcohol having a branched chain and 12 to 19 carbon atoms that is used as component (B) in the oil agent for cosmetics of the present invention is not particularly limited as long as it can be used as an oil agent in cosmetics, and one having 14 to 19 carbon atoms is preferred, and one having 17 to 18 carbon atoms is more preferred.

Specific examples of component (B) include 2-butyl-1-octanol, 2-pentyl-1-nonanol, 2-hexyl-1-decanol, 14-methyl-1-pentadecanol, 2-pentyl-1-dodecanol, 2-octyl-1-nonanol, 16-methyl-1-heptadecanol, 2-octyl-1-decanol, 4-methyl-1-octadecanol and the like. Among these, 2-pentyl-1-nonanol, 2-hexyl-1-decanol, 14-methyl-1-pentadecanol, 2-pentyl-1-dodecanol, 2-octyl-1-nonanol, 16-methyl-1-heptadecanol, 2-octyl-1-decanol, and 4-methyl-1-octadecanol are preferred, and 2-pentyl-1-dodecanol, 2-octyl-1-nonanol, 16-methyl-1-heptadecanol, and 2-octyl-1-decanol are more preferred.

In the present invention, one kind of the above-mentioned component (B) may be used alone, or two or more kinds thereof may be used in combination.

As the above-mentioned component (B), commercially available products provided by various companies for use in cosmetics can be used.

The content of component (B) in the oil agent for cosmetics of the present invention is 1% by mass to 30% by mass.

When the content of component (B) is less than 1% by mass, a sense of adsorption after application may become poor. From the aspect of the sense of adsorption after application, the content of component (B) is preferably 3% by mass or more, more preferably 5% by mass or more.

When the content of component (B) exceeds 30% by mass, the feeling of thickness when applied and the feeling of penetration into the skin may become poor. From the aspects of the feeling of thickness when applied and the feeling of penetration into the skin, the content of component (B) is preferably 25% by mass or less, more preferably 22% by mass or less, further preferably 20% by mass or less.

The total content of the component (A) and component (B) in the oil agent for cosmetics of the present invention is preferably 76% by mass to 100% by mass, more preferably 81% by mass to 100% by mass, further preferably 85% by mass to 100% by mass.

The oil agent for cosmetics of the present invention may contain an oil agent other than component (A) and component (B) and a lipophilic additive, as long as the characteristics of the present invention are not impaired.

The oil agent other than component (A) and component (B) is not particularly limited as long as it shows compatibility with component (A) and component (B). From the aspects of the feeling of thickness when applied and a sense of adsorption after application, triglyceride which is described later as component (E) is preferably contained.

As the lipophilic additive, lipophilic antioxidants, antiseptics, ultraviolet absorbers, and the like can be mentioned.

The oil agent for cosmetics of the present invention can be produced by further adding lipophilic oil agents and lipophilic additives as necessary to the above-mentioned component (A) and component (B), and uniformly mixing them.

The oil agent for cosmetics of the present invention can be preferably used as an oily starting material for cosmetics. The cosmetic for which the oil agent for cosmetics of the present invention can be used is not particularly limited as long as it is in a form capable of containing an oily component. Examples include oily cosmetics such as beauty oil, cleansing oil, hair oil, and the like; emulsion cosmetics for skin or hair, such as milky lotion, skin cream, hair cream, and the like; emulsified makeup cosmetics such as milky liquid foundation, creamy foundation, and the like; and the like. It can be particularly preferably used in the below-mentioned emulsion cosmetics of the present invention.

The oil agent for cosmetics of the present invention can be used as a texture improving agent in cosmetics. When it is used particularly in emulsion cosmetics, a feeling of thickness when applied to the skin can be imparted more, and the effects of superior feeling of penetration into the skin in a high temperature and high humidity state, and improvement of an unpleasant sense of adsorption after application can be exhibited more.

The above-mentioned cosmetics containing the oil agent for cosmetics of the present invention can be produced by a method generally employed, by using, according to each form, general components for cosmetics such as water, lower alcohol such as ethanol and the like, an oil agent other than the above-mentioned component (A) and component (B), surfactant, humectant, thickener, hair conditioning agent, antioxidant, stabilizer, antiseptic, pearling agent, pH adjuster, ultraviolet absorber, hydrotropic agent, pigment, dye, antimicrobial agent, anti-inflammatory agent, astringent, algefacient, flavor, plant extract, and the like as necessary.

The present invention also provides emulsion cosmetics.

The emulsion cosmetics of the present invention contain the oil agent for cosmetics of the present invention, (C) divalent to hexavalent polyhydric alcohol having 3 to 6 carbon atoms, and (D) non-ionic surfactant having an HLB value of 10 to 18.

The emulsion cosmetics of the present invention contain the above-mentioned oil agent for cosmetics of the present invention as a texture improving agent.

The content of the oil agent for cosmetics of the present invention in the emulsion cosmetics of the present invention is preferably 1% by mass to 60% by mass, more preferably 1% by mass to 50% by mass, further preferably 1% by mass to 40% by mass. When the content of the oil agent for cosmetics of the present invention is less than 1% by mass, the texture improving effect may not be exhibited sufficiently. When the content of the oil agent for cosmetics of the present invention exceeds 60% by mass, the feeling of penetration into the skin in a high temperature and high humidity state may become insufficient, and an unpleasant sense of adsorption may occur after application.

The divalent to hexavalent polyhydric alcohol having 3 to 6 carbon atoms which is contained as component (C) in the emulsion cosmetics of the present invention is not particularly limited as long as it is a starting material that can be generally blended in cosmetics. Examples include divalent alcohol such as propylene glycol (1,2-propanediol), 1,3-propanediol, dipropylene glycol, 1,3-butyleneglycol, 1,2-pentanediol, 1,2-hexanediol and the like; trivalent alcohol such as glycerol and the like; diglycerol; tetoritol such as erythritol, threitol and the like; pentitol such as arabinitol, xylitol, ribitol and the like; hexitol such as iditol, galactitol, sorbitol, mannitol and the like; and the like. from the aspect of a sense of adsorption after application, a divalent alcohol or trivalent alcohol having 3 to 6 carbon atoms is more preferred, and a divalent alcohol having 3 to 6 carbon atoms is further preferred.

Specific examples of preferred component (C) include propylene glycol, dipropylene glycol, 1,3-propanediol, 1,3-butyleneglycol, glycerol, diglycerol, and the like.

In the present invention, one kind of the above-mentioned component (C) may be used alone, or two or more kinds thereof may be used in combination.

As the above-mentioned component (C), commercially available products provided by various companies for use in cosmetics can be used.

From the aspects of a feeling of penetration into the skin and a sense of adsorption after application, a ratio of the total content of the component (A) and the component (B) to the content of the component (C) [{ (A)+(B)}/(C)] is 0.05 to 30, preferably 0.1 to 25, more preferably 0.2 to 20, further preferably 0.2 to 10, in a mass ratio.

The content of component (C) in the emulsion cosmetics of the present invention is preferably 1% by mass to 20% by mass, more preferably 1% by mass to 15% by mass.

The non-ionic surfactant having an HLB (Hydrophilic-Lipophilic Balance) value of 10 to 18, which is used as component (D) in the emulsion cosmetics of the present invention, is not particularly limited as long as it is a non-ionic surfactant having an HLB value of 10 to 18 and generally used for cosmetics. Examples include polyoxyethylene alkyl or alkenylether having a polyether chain as a hydrophilic group, such as polyoxyethylene (9E.O.)lauryl ether, polyoxyethylene (10E.O.)oleyl ether, and the like; polyoxyethylene polyoxypropylene glycol such as polyoxyethylene polyoxypropylene glycol (160 E.O.) (30P.O.) and the like; polyoxyethylene polyoxypropylene alkylether such as polyoxyethylene (20E.O.)polyoxypropylene (8P.O.)cetyl ether, polyoxyethylene (30E.O.)polyoxypropylene (6P.O.)decyltetradecylether, and the like; polyoxyethylene hydrogenated castor oil such as polyoxyethylene (40E.O.) hydrogenated castor oil, polyoxyethylene (60E.O.) hydrogenated castor oil, and the like; polyoxyethylene castor oil such as polyoxyethylene (30E.O.)castor oil, polyoxyethylene (50E.O.)castor oil, and the like; polyethylene glycol fatty acid ester such as polyethylene glycol (25E.O.) monostearate and the like; polyoxyethylene glycerol fatty acid ester such as polyoxyethylene (15E.O.)glyceryl monostearate and the like; polyoxyethylene sorbitan fatty acid ester such as polyoxyethylene (20E.O.) sorbitan monolaurate, polyoxyethylene (20E.O.)sorbitan monostearate, polyoxyethylene (20E.O.)sorbitan monoisostearate, polyoxyethylene (20E.O.)sorbitan monooleate, and the like; polyoxyethylene sorbitol fatty acid ester such as polyoxyethylene (60E.O.)sorbitol tetrastearate, polyoxyethylene (60E.O.)sorbitol tetraoleate, and the like; polyglycerol fatty acid ester having a polyglycerol chain as a hydrophilic group, such as pentaglyceryl monolaurate, pentaglyceryl monostearate, hexaglyceryl monolaurate, decaglyceryl monolaurate, decaglyceryl monomyristate, decaglyceryl monostearate, decaglyceryl distearate, and the like; and the like.

Component (D) is used as an emulsifier in the emulsion cosmetics of the present invention, and one kind thereof may be used alone, or two or more kinds thereof may be used in combination.

As the above-mentioned component (D), commercially available products provided by various companies as non-ionic surfactants for cosmetics can be used.

From the aspect of more stable emulsification of the oil agent for cosmetics of the present invention, a component (D) having an HLB value of 10 to 17 is preferred, and a component (D) having an HLB value of 10 to 16 is more preferred. The HLB value of a non-ionic surfactant can be calculated by the Griffin method.

From the aspects of a feeling of thickness when applied to the skin, and a sense of adsorption after application, polyoxyethylene sorbitan fatty acid ester and polyglycerol fatty acid ester having an HLB value of 10 to 18 are preferably used, and polyglycerol fatty acid ester is more preferably used. From the aspect of a sense of adsorption after application, the carbon number of the fatty acid constituting the aforementioned polyoxyethylene sorbitan fatty acid ester or polyglycerol fatty acid ester is preferably 10 to 20, more preferably 12 to 18.

The average degree of polymerization of the polyglycerol constituting the above-mentioned polyglycerol fatty acid ester is not particularly limited as long as it has HLB value of 10 to 18 and can stably emulsify the oil agent for cosmetics of the present invention. It is preferably 2 to 10, more preferably 3 to 10.

Examples of the above-mentioned polyoxyethylene sorbitan fatty acid ester or polyglycerol fatty acid ester include a monoester form, a diester form, a triester form, and the like. The polyoxyethylene sorbitan fatty acid ester and the polyglycerol fatty acid ester are not particularly limited as long as they have an HLB value of 10 to 18 and can stably emulsify the oil agent for cosmetics of the present invention. A monoester form and a diester form are preferably used, and a monoester form is more preferably used.

From the aspect of emulsion stability, the content of component (D) in the emulsion cosmetics of the present invention is preferably 0.1% by mass to 10% by mass, more preferably 0.1% by mass to 5% by mass.

The emulsion cosmetics of the present invention may further contain triglyceride as component (E) in addition to the above-mentioned oil agent for cosmetics of the present invention, component (C) and component (D). The emulsion cosmetic of the present invention more preferably contains component (E) because the effects of improving a feeling of thickness when applied to the skin and an unpleasant sense of adsorption after application are improved.

Examples of the triglyceride used as the component (E) in the emulsion cosmetics of the present invention include a triglyceride containing one or more higher fatty acids such as caproic acid, caprylic acid, capric acid, 2-ethylhexanoic acid, lauric acid, tridecanoic acid, isotridecanoic acid, myristic acid, palmitic acid, isopalmitic acid, stearic acid, isostearic acid, eicosanoic acid, behenic acid, tetracosanoic acid, myristoleic acid, palmitoleic acid, oleic acid, elaidic acid, erucic acid, linoleic acid, linolenic acid, coconut oil fatty acid, palm kernel oil fatty acid, hydrogenated palm kernel oil fatty acid, palm oil fatty acid, castor oil fatty acid, hydrogenated castor oil fatty acid and the like as constituent fatty acids, animal and plant fats and oils such as olive oil, macadamia nut oil, sunflower oil, palm oil, palm kernel oil, safflower oil, castor oil, hydrogenated castor oil, coconut oil, camellia oil, cacao butter, shea butter and the like, and the like. Among these, olive oil, sunflower oil, and tri(caprylic acid/capric acid) glyceride are preferably used. One kind of these components (E) may be used alone, or two or more kinds thereof may be used in combination.

As the above-mentioned component (E), commercially available products provided by various companies for use in cosmetics can be used.

The content of component (E) in the emulsion cosmetics of the present invention is preferably 0.1% by mass to 20% by mass, more preferably 0.1% by mass to 10% by mass.

The emulsion cosmetics of the present invention may contain, where necessary, other components generally used for cosmetics; for example, solvents such as water, lower alcohol, and the like; oil agents such as hydrocarbon oil, higher fatty acid, straight chain higher alcohol, silicone oil, ester oil, and the like; additives such as surfactant other than the above-mentioned component (D), humectant, thickener, antioxidant, stabilizer, antiseptic, pearling agent, pH adjuster, ultraviolet absorber, hydrotropic agent, pigment, dye, antimicrobial agent, anti-inflammatory agent, astringent, algefacient, flavor, plant extract, and the like, as long as the characteristics of the present invention are not impaired.

The emulsion cosmetics of the present invention can be produced by emulsifying by a general emulsifying means such as a method in which water, hydrophilic additives, and the like are added as necessary to the above-mentioned component (C) and component (D) to make a homogeneous mixture, which is then heated to prepare an aqueous phase, other oily components such as component (E) and lipophilic additives are added as necessary to the oil agent for cosmetics of the present invention to make a homogeneous mixture, which is then heated to prepare an oil phase, and the oil phase is added and mixed with the aqueous phase to emulsify the mixture, and a method in which water, hydrophilic additives, other oily components such as component (E) and the like, and lipophilic additives are add as necessary to the oil agent for cosmetics of the present invention, the above-mentioned component (C) and component (D), and they are mixed and heated, and a shear force is applied by a homomixer and the like to emulsify the mixture.

The emulsion cosmetics of the present invention can be preferably provided as oil-in-water emulsion cosmetics, and can be provided as skin cosmetics such as milky lotion, massage cream, face cream, moisturizing cream, and the like, body cosmetics such as hand cream, foot cream, body cream, and the like, sunscreen cosmetics such as sunscreen milky lotion, sunscreen cream, and the like, primers such as primer lotion, primer cream, and the like, makeup cosmetics such as liquid foundation, creamy foundation, and the like, and the like.

The emulsion cosmetic of the present invention is superior in a feeling of thickness when applied to the skin in a high temperature and high humidity state and a feeling of penetration into the skin in a high temperature and high humidity state, and further causes less unpleasant sense of adsorption after application.

Therefore, the emulsion cosmetic of the present invention is suitable for use on wet skin after bathing and the skin in a high temperature and high humidity state after towel drying, and also affords superior operability of a liquid crystal display screen.

Furthermore, the present invention provides cosmetics containing an inorganic powder (hereinafter also to be referred to as "the powder-containing cosmetics of the present invention" in the present specification).

The powder-containing cosmetic of the present invention contains (A) primary alcohol having a branched chain and 20 to 30 carbon atoms, (B) primary alcohol having a branched chain and 12 to 19 carbon atoms, and (F) inorganic powder having an average particle size of 10 nm to 100 µm.

The primary alcohol having a branched chain and 20 to 30 carbon atoms which is contained as component (A) in the powder-containing cosmetic of the present invention is as described above for the oil agent for cosmetic of the present invention.

The content of component (A) in the powder-containing cosmetic of the present invention is 0.5% by mass to 60% by mass.

When the content of component (A) is less than 0.5% by mass, the effect of suppressing a feeling of dryness after application and a sense of friction when applied is not obtained sufficiently, and a feeling of thickness when applied to the wet skin may be insufficient.

From the aspects of the effect of suppressing a feeling of dryness after application and a sense of friction when applied, and a feeling of thickness when applied to the wet skin, the content of component (A) is preferably 1% by mass or more, more preferably 2.5% by mass or more.

When the content of component (A) exceeds 60% by mass, the effect of suppressing a feeling of dryness after application and a sense of friction when applied may be insufficient, and further, the feeling of blending into the wet skin may become poor.

From the aspects of the texture when applied to the skin such as a feeling of dryness and a sense of friction, and improvement of the feeling of blending into the wet skin, the content of component (A) is preferably 40% by mass or less, more preferably 30% by mass or less, further preferably 20% by mass or less, particularly preferably 15% by mass or less.

The primary alcohol having a branched chain and 12 to 19 carbon atoms which is contained as component (B) in the powder-containing cosmetic of the present invention is as described above for the oil agent for cosmetic of the present invention.

The content of component (B) in the powder-containing cosmetic of the present invention is 0.01% by mass to 30% by mass.

When the content of component (B) is less than 0.01% by mass, the effect of suppressing a feeling of dryness after application and a sense of friction when applied is not obtained sufficiently, and a feeling of blending into the skin when applied to the wet skin may be insufficient.

From the aspects of the effect of suppressing a feeling of dryness after application and a sense of friction when applied, and a feeling of blending into the skin when applied to the wet skin, the content of component (B) is preferably 0.05% by mass or more, more preferably 0.1% by mass or more.

When the content of component (B) exceeds 30% by mass, the effect of suppressing a feeling of dryness after application and a sense of friction when applied becomes insufficient, and further, the feeling of thickness when applied to the wet skin and the feeling of blending into the wet skin may become poor.

From the aspects of the effect of suppressing a feeling of dryness after application to the skin and a sense of friction when applied to the skin, as well as the feeling of thickness when applied to the wet skin and the feeling of blending into the wet skin, the content of component (B) is preferably 20% by mass or less, more preferably 10% by mass or less.

The content ratio of component (A) to component (B) [(A)/(B)] in the powder-containing cosmetic of the present invention is 2.3 to 99 in a mass ratio.

When the aforementioned ratio [(A)/(B)] is less than 2.3, the effect of suppressing a feeling of dryness after application and a sense of friction when applied may be insufficient, and further, the feeling of thickness when applied to the wet skin and the feeling of blending into the wet skin may become poor.

When the aforementioned ratio [(A)/(B)] exceeds 99, the effect of suppressing a feeling of dryness after application and a sense of friction when applied may be insufficient, and further, the feeling of blending into the wet skin may become poor.

From the aspects of the texture when applied to the skin such as a feeling of dryness and a sense of friction, and improvement of a feeling of thickness when applied to the wet skin and a feeling of blending into the wet skin, the aforementioned ratio [(A)/(B)] is preferably 3 or more, more preferably 4 or more. In addition, the aforementioned ratio [(A)/(B)] is preferably 30 or less, more preferably 20 or less, further preferably 10 or less.

The total content of component (A) and component (B) in the powder-containing cosmetic of the present invention is 1% by mass to 60% by mass, preferably 3% by mass or more. The total content of component (A) and component (B) is preferably 50% by mass or less, more preferably 40% by mass or less, further preferably 30% by mass or less, particularly preferably 20% by mass or less.

When the total content of component (A) and component (B) in the powder-containing cosmetic of the present invention is less than 1% by mass, the effect of improving the texture when applied to the skin may not be exhibited sufficiently.

When the total content of component (A) and component (B) exceeds 60% by mass, the effect of suppressing a sense of friction when applied may become insufficient, and the feeling of blending into the wet skin may become poor.

As the component (A) and component (B) in the powder-containing cosmetic of the present invention, those prepared in advance as the above-mentioned oil agent for cosmetic of the present invention can also be used.

The inorganic powder to be contained as component (F) in the powder-containing cosmetic of the present invention is not particularly limited as long as it is generally used as an inorganic pigment in cosmetics.

Specifically, for example, inorganic extender pigment (e.g., silica, talc, kaolin, mica, aluminum oxide, calcium carbonate, magnesium carbonate, barium sulfate, etc.); inorganic white pigment (e.g., titanium oxide, zinc oxide, zirconium oxide, etc.); inorganic red color pigment (e.g., red iron oxide (red ferric oxide), iron (II) titanium oxide, etc.); inorganic brown color pigment (e.g., γ-ferric oxide, etc.); inorganic yellow color pigment (e.g., yellow iron oxide (ferric hydroxy oxide and ferric hydroxide), yellow ocher, etc.); inorganic black color pigment (e.g., black iron oxide (triiron tetraoxide), titanium lower oxide, carbon black, etc.); inorganic violet color pigment (e.g., manganese violet, cobalt violet (cobalt phosphate, hydrous ammonium cobalt(II) phosphate, etc.), etc.); inorganic green color pigment (e.g., chrome oxide, chrome hydroxide, cobalt titanate, etc.); inorganic blue color pigment (e.g., cobalt blue (aluminum cobalt oxide), ultramarine blue (complex sodium aluminum sulfosilicates), iron blue (iron(III) ferrocyanide, ammonium iron (III) hexacyanoferrate (II), etc.); pearl pigment (e.g., mica titanium (titanium oxide coated mica), titanium oxide coated bismuth oxychloride, titanium oxide coated talc, bismuth oxychloride, etc.); colored pearl pigment (e.g., iron oxide coated mica, iron oxide coated mica titanium, carbon black coated mica titanium, black iron oxide coated mica titanium, iron hydroxide coated mica titanium, iron blue coated mica titanium, chrome hydroxide coated mica titanium, chrome oxide coated mica titanium, cobalt titanate coated mica titanium, titanium lower oxide coated mica, etc.), metal powder pigment (e.g., aluminum powder, copper powder, etc.), and the like can be mentioned.

As component (F) in the present invention, a powder obtained by subjecting the inorganic pigments mentioned above to a surface treatment with an inorganic compound such as silica, aluminum oxide, zirconium oxide and the like, or an organic compound such as silicone compound, fluorine compound (perfluoroalkylphosphoric acid ester, perfluoroalkylphosphate, perfluoroalkylsilane, etc.), silane coupling agent, N-acyl amino acid (N-stearoyl-L-glutamic acid, etc.), metal soap (aluminum stearate, etc.), amino acid, and the like can also be used.

For the purpose of the present invention, extender pigments such as silica, talc, mica, and the like; white pigments such as titanium oxide, zinc oxide, and the like; iron oxide (red iron oxide, yellow iron oxide, black iron oxide, etc.) and the like; and aluminum oxide-treated powders, hydrophobic treating powders (silicone-treated powder, N-acyl amino acid-treated powder, metal soap-treated powder, etc.) of these are preferably used.

The average particle size of the inorganic powder contained as component (F) in the powder-containing cosmetic of the present invention is 10 nm to 100 µm, preferably 10 nm to 2 µm, more preferably 10 nm to 1 µm, further preferably 10 nm to 300 nm, particularly preferably 10 nm to 50 nm.

As used herein, the "average particle size" refers to the average particle size of the primary particles of the inorganic powder, and is measured by dynamic light scattering or laser diffraction scattering method.

In the present invention, one kind of the above-mentioned component (F) may be used alone, or two or more kinds thereof may be used in combination.

As the above-mentioned component (F), commercially available products provided by various companies for use in cosmetics can be used.

The content of component (F) in the powder-containing cosmetic of the present invention is 2% by mass to 50% by mass.

When the content of component (F) is less than 2% by mass, a feeling of thickness when applied to the wet skin and a feeling of blending into the wet skin tend to be not achieved.

From the aspects of a feeling of thickness when applied to the skin and a feeling of blending into the wet skin, the content of component (F) in the powder-containing cosmetic of the present invention is preferably 2.5% by mass or more, more preferably 5% by mass or more, further preferably 10% by mass or more, particularly preferably 18% by mass or more.

On the other hand, when the content of component (F) exceeds 50% by mass, a feeling of dryness after application or a sense of friction when applied may occur.

From the aspects of a feeling of dryness after application and a sense of friction when applied, the content of component (F) is preferably 40% by mass or less, more preferably 35% by mass or less.

Furthermore, the powder-containing cosmetic of the present invention may contain, where necessary, other components generally used for cosmetics, for example, solvents such as water, lower alcohol, and the like; oil agents other than component (A) and component (B) such as hydrocarbon oil, higher fatty acid, wax, silicone oil, ester oil, and the like; additives such as surfactant, emulsifier, dispersing agent, suspending agent, humectant, thickener, antioxidant, stabilizer, antiseptic, pearling agent, pH adjuster, ultraviolet absorber, hydrotropic agent, organic pigment, dye, antimicrobial agent, anti-inflammatory agent, astringent, algefacient, flavor, plant extract, and the like, as long as the characteristics of the present invention are not impaired.

The powder-containing cosmetic of the present invention can be produced by a general production method that is adopted according to the form of the cosmetic.

For example, emulsion cosmetics can be prepared by a machine emulsion method including mixing the above-mentioned (A), (B), component (F), additives added as necessary, and water, applying a shear force by using a homo mixer, a homogenizer, a high-pressure homogenizer, or the like to emulsify the mixture, an Agent-in-water method including dispersing the above-mentioned component (F) in an aqueous phase containing hydrophilic additives added as necessary and an emulsifier, and adding under stirring an oil phase containing the above-mentioned component (A) and component (B), and lipophilic additives added as necessary, an Agent-in-oil method including dispersing the above-mentioned component (F) in an oil phase containing the above-mentioned component (A) and component (B), lipophilic additives added as necessary and an emulsifier, and adding under stirring an aqueous phase containing hydrophilic additives added as necessary, and the like.

The powder-containing cosmetic of the present invention can be provided as, for example, an oily cosmetic or an oil-in-water or water-in-oil emulsion cosmetic, and can be provided as skin cosmetics such as milky lotion, massage cream, face cream, moisturizing cream, and the like, body cosmetics such as hand cream, foot cream, body cream, and the like, sunscreen cosmetics such as sunscreen milky lotion, sunscreen cream, and the like, primers such as primer lotion, primer cream, and the like, base makeup cosmetics such as liquid foundation, creamy foundation, and the like, point makeup cosmetics such as eyecolor, blush, lip color, and the like, and the like.

The powder-containing cosmetic of the present invention can contain an inorganic powder having a coloration effect or an UV defense effect. Therefore, it can be particularly preferably provided as sunscreen cosmetics, primer, base makeup cosmetics, and the like.

The powder-containing cosmetic of the present invention suppresses a feeling of dryness and a sense of friction that occur due to the inclusion of inorganic powder, and even when applied to wet skin, it can simultaneously achieve a feeling of thickness and a feeling of blending into the skin.

The powder-containing cosmetic of the present invention is therefore suitable for use on the skin after face washing and skin care, wet skin after bathing, and the like.

### [Example]

The present invention is described in more detail below based on examples, but the present invention is not limited to these examples.

Examples of the oil agent for cosmetic of the present invention are shown.

### [Examples 1 - 7 and Comparative Examples 1 - 5] oil agent for cosmetics

According to the compositions shown in Table 1, the components in Table 1 were mixed uniformly to prepare the oil agents for cosmetics of Examples 1 to 7 and the oil agents for cosmetics of Comparative Examples 1 to 5. Note that commercially available products provided for use in cosmetics were used as the component (A), component (A'), component (B) and component (B') in Table 1.

The oil agents for cosmetics of Examples 1 to 7 and Comparative Example 1 to 5 were evaluated for a feeling of thickness when applied to the skin in a high temperature and high humidity state and a feeling of penetration into the skin in a high temperature and high humidity state, as shown below, and the evaluation results are also shown in Table 1.

### (1) Feeling of thickness when applied to the skin in a high temperature and high humidity state

20 female panelists (22 years old to 40 years old) soaked their forearms in 40°C hot water for 5 min, then lightly removed the moisture with a towel. Immediately thereafter, they applied about 1 g of each of the oil agents for cosmetics of Examples 1 to 7 and Comparative Examples 1 to 5 to the inner side of their forearms and evaluated the feeling of thickness when blended with their fingers, according to the following evaluation criteria. The total score of the 20 panelists was calculated, and the feeling of thickness when applied to the skin in a high temperature and high humidity state was determined based on the total score and according to the following determination criteria. An oil agent for cosmetics that received a rating of "○" or "⊙" was evaluated as an oil agent for cosmetics which has a good feeling of thickness when applied to the skin in a high temperature and high humidity state.

### <Evaluation criteria>

2 points: when applied to the skin, feeling of thickness was felt very good
1 point: when applied to the skin, feeling of thickness was felt slightly good
0 point: when applied to the skin, feeling of thickness was felt slightly bad

### <Determination criteria>

⊙: the total score is 35 points or more
○: the total score is 30 points or more and 34 points or less
Δ: the total score is 20 points or more and 29 points or less
×: the total score is 19 points or less

### (2) Feeling of penetration into the skin in a high temperature and high humidity state

20 female panelists (22 years old to 40 years old) soaked their forearms in 40°C hot water for 5 min, then lightly removed the moisture with a towel. Immediately thereafter, they applied about 1 g of each of the oil agents for cosmetics of Examples 1 to 7 and Comparative Examples 1 to 5 to the inner side of their forearms and evaluated the feeling of penetration when blended with their fingers, according to the following evaluation criteria. The total score of the 20 panelists was calculated, and the feeling of penetration into the skin in a high temperature and high humidity state was determined based on the total score and according to the following determination criteria. An oil agent for cosmetics that received a rating of "○" or "⊙" was evaluated as an oil agent for cosmetics which has a good feeling of penetration into the skin in a high temperature and high humidity state.

### <Evaluation criteria>

2 points: when applied to the skin, feeling of penetration was felt very good
1 point: when applied to the skin, feeling of penetration was felt slightly good
0 point: when applied to the skin, feeling of penetration was felt slightly bad

### <Determination criteria>

⊙: the total score is 35 points or more
O: the total score is 30 points or more and 34 points or less
Δ: the total score is 20 points or more and 29 points or less
×: the total score is 19 points or less

**[Table 1]**

| component | | Example | | | | | | | Comparative Example | | | | |
|---|---|---|---|---|---|---|---|---|---|---|---|---|---|
| | | 1 | 2 | 3 | 4 | 5 | 6 | 7 | 1 | 2 | 3 | 4 | 5 |
| (A) | 2-octyl-1-dodecanol | 80 | | 40 | 95 | 80 | 90 | 75 | 100 | | 55 | 40 | 80 |
| | 2-decyl-1-tetradecanol | | 80 | | | | | | | | | | |
| | 2-dodecyl-1-hexadecanol | | | 40 | | | | | | | | | |
| (A') | behenyl alcohol | | | | | | | | | | | 40 | |
| (B) | 2-pentyl-1-nonanol | 20 | | | | | | 25 | | | | | |
| | 2-pentyl-1-dodecanol | | | | 5 | | | | | | | | |
| | 16-methyl-1-heptadecanol | | 20 | 20 | | 20 | | | | 100 | 45 | 20 | |
| | 2-octyl-1-decanol | | | | | | 10 | | | | | | |
| (B') | cetanol | | | | | | | | | | | | 20 |
| total | | 100 | 100 | 100 | 100 | 100 | 100 | 100 | 100 | 100 | 100 | 100 | 100 |
| evaluation item | feeling of thickness when applied to skin in high temperature and high humidity state | ○ (30) | ⊙ (38) | ⊙ (38) | ⊙ (38) | ⊙ (38) | ⊙ (38) | ○ (31) | Δ (29) | × (19) | Δ (21) | ○ (32) | ○ (31) |
| | feeling of penetration into skin in high temperature and high humidity state | ⊙ (38) | ○ (33) | ○ (30) | ⊙ (35) | ⊙ (37) | ⊙ (35) | ○ (34) | × (16) | Δ (26) | Δ (22) | × (15) | × (18) |

| | | | | | | | | | | | | | |
|---|---|---|---|---|---|---|---|---|---|---|---|---|---|
| *; The numerical value for each component in the table indicates the content (% by mass) of the component, and a blank column indicates that the component is not contained. **; The numerical value in parenthesis in the evaluation results indicates the total score. | | | | | | | | | | | | | |

As shown in Table 1, the oil agents for cosmetics of Examples 1 to 7 were all evaluated to be oil agents for cosmetics having a good feeling of thickness when applied to the skin in a high temperature and high humidity state, and a good feeling of penetration into the skin in a high temperature and high humidity state.

In particular, the oil agents for cosmetics of Examples 4 **to 6,** that respectively contain 95% by mass, 80% by mass, and 90% by mass of 2-octyl-1-dodecanol as component (A) and **2-**pentyl-1-dodecanol, 16-methyl-1-heptadecanol, and 2-octyl-1-decanol as component (B), were evaluated to be very good in both a feeling of thickness when applied to the skin in a high temperature and high humidity state, and a feeling of penetration into the skin in a high temperature and high humidity state.

On the other hand, the oil agent for cosmetics of Comparative Example 1, which does not contain component (B), was evaluated as having an insufficient feeling of thickness when applied to the skin in a high temperature and high humidity state, and having a bad feeling of penetration into the skin in a high temperature and high humidity state.

The oil agent for cosmetics of Comparative Example 2, which does not contain component (A), was evaluated as having a bad feeling of thickness when applied to the skin in a high temperature and high humidity state, an insufficient feeling of penetration into the skin in a high temperature and high humidity state.

The oil agent for cosmetics of Comparative Example 3, in which the content of component (A) is less than 70% by mass and the content of component (B) exceeds 30% by mass, was evaluated to be insufficient in the feeling of thickness when applied to the skin in a high temperature and high humidity state and the feeling of penetration into the skin in a high temperature and high humidity state.

The oil agent for cosmetics of Comparative Example 4, in which a part of component (A) was replaced with behenyl alcohol (linear primary alcohol having 22 carbon atoms) which is component (A'), and the content of component (A) was less than 70% by mass, and the oil agent for cosmetics of Comparative Example 5, which contained cetanol (linear primary alcohol having 16 carbon atoms) which is component (B') instead of component (B), were evaluated as having a bad feeling of penetration into the skin in a high temperature and high humidity state.

Next, examples of the emulsion cosmetic of the present invention are shown.

### [Examples 8 - 17, Comparative Examples 6 - 13] Emulsion cosmetics

According to the compositions shown in Tables 2 and 3, emulsion cosmetics containing the oil agents for cosmetics of Examples 1 to 7 and Comparative Examples 1 to 5 were prepared by the following method and used as Examples 8 to 17 and Comparative Examples 6 to 13. The common addition components in Tables 2 and 3 are shown in Table 4. As component (C), component (D), and component (E) in Tables 2, 3 and each component shown in Table 4, commercially available products provided for use in cosmetics were used.

### <Preparation method>

Each of the oil agent for cosmetics of Examples 1 to 7 and Comparative Examples 1 to 5, component (C), component (D), component (E), common addition component and water in Tables 2, 3 were mixed, heated to 75°C to 80°C, a shear force was applied using a homomixer to mix and emulsify at that temperature, and then the mixture was cooled to prepare emulsion cosmetics.

Each of the emulsion cosmetics of Examples 8 to 17 and Comparative Examples 6 to 13 was evaluated for (1) a feeling of thickness when applied to the skin in a high temperature and high humidity state, (2) a feeling of penetration into the skin in a high temperature and high humidity state, (3) an unpleasant sense of adsorption after application.

The feeling of thickness when applied to the skin in a high temperature and high humidity state and the feeling of penetration into the skin in a high temperature and high humidity state were evaluated in the same manner as in the above-mentioned oil agent for cosmetics. The unpleasant sense of adsorption after application was evaluated by the method shown in the following.

### (3) unpleasant sense of adsorption after application

20 female panelists (22 years old to 40 years old) applied about 1 g of each of the emulsion cosmetics of Example 8 to 17 and Comparative Example 6 to 13, operated liquid crystal display screens with fingers immediately after the application, and evaluated the unpleasant sense of adsorption felt at that time, according to the following evaluation criteria. The total score of the 20 panelists was calculated, and the unpleasant sense of adsorption after application was determined based on the total score and according to the following determination criteria. An emulsion cosmetic that received a rating of "○" or "⊙" was evaluated as an emulsion cosmetic with less unpleasant sense of adsorption.

### <Evaluation criteria>

2 points: when liquid crystal display screen was touched, fingerprint was not visible, a sense of finger being caught on the display screen was absent, and no unpleasant sense of adsorption was felt
1 point: when liquid crystal display screen was touched, fingerprint was slightly visible, a sense of finger being slightly caught on the display screen was present, and a slightly unpleasant sense of adsorption was felt
0 point: when liquid crystal display screen was touched, fingerprint was visible, a sense of finger being caught on the display screen was present, and an unpleasant sense of adsorption was felt

### <Determination criteria>

⊙: the total score is 35 points or more
O: the total score is 30 points or more and 34 points or less
Δ: the total score is 20 points or more and 29 points or less
×: the total score is 19 points or less

The evaluation results are also shown in Tables 2 and 3.

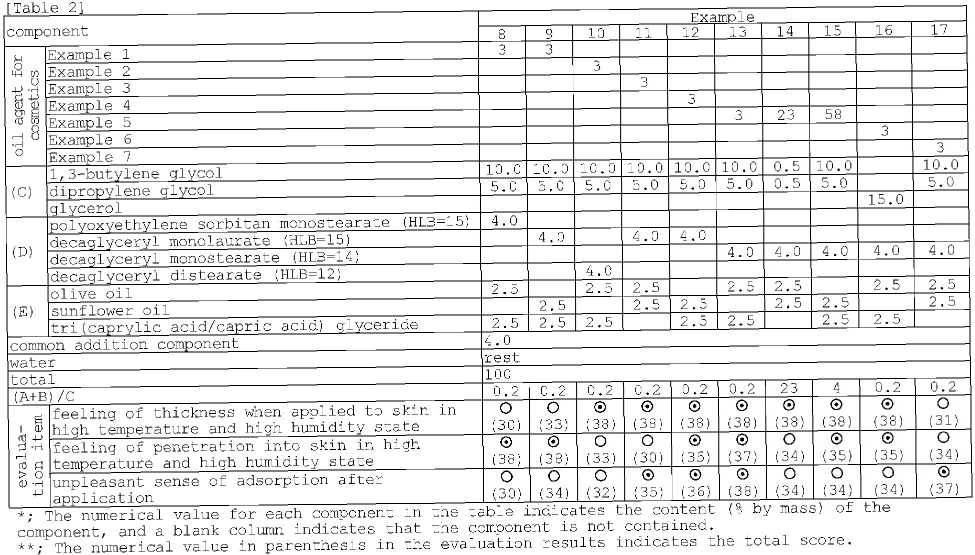

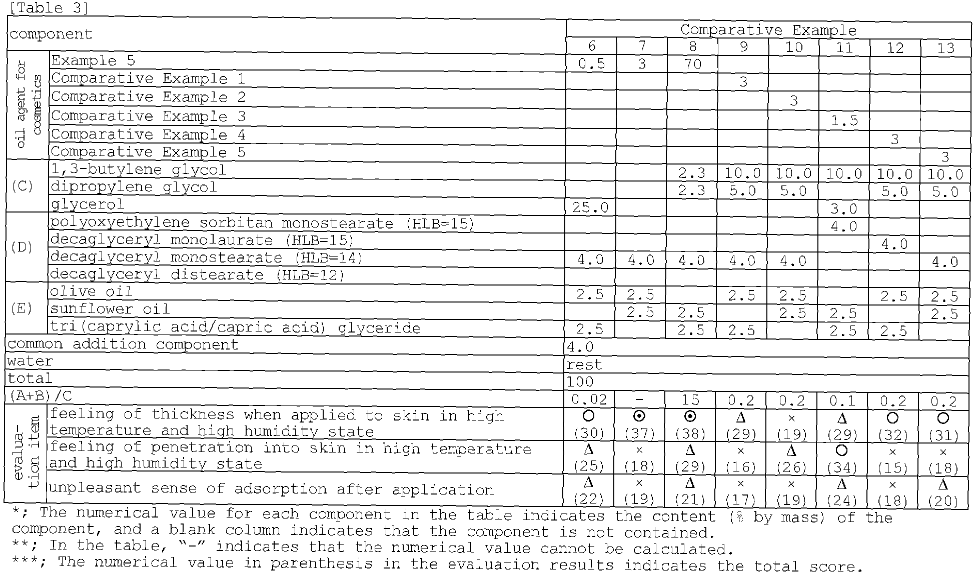

**[Table 4]**

| component | content (% by mass) |
|---|---|
| carboxyvinyl polymer | 0.2 |
| beeswax | 2.5 |
| glyceryl stearate | 1.0 |
| phenoxyethanol | 0.3 |
| total | 4.0 |

As shown in Table 2, the emulsion cosmetics of Examples 8 to 17 were all evaluated to be emulsion cosmetics having a good feeling of thickness when applied to the skin in a high temperature and high humidity state and a good feeling of penetration into the skin in a high temperature and high humidity state, with less unpleasant sense of adsorption after application.

In particular, each of the emulsion cosmetics of Example 12 and 13 containing 3% by mass of the oil agent for cosmetics of Example 4 or 5, containing 1,3-butylene glycol and dipropylene glycol as component (C), and having a ratio of the total content of the component (A) and the component (B) to the content of the component (C) [{(A)+(B)}/(C)] of 0.2 was evaluated as having a very good feeling of thickness when applied to the skin in a high temperature and high humidity state and a very good feeling of penetration into the skin in a high temperature and high humidity state, with very little unpleasant sense of adsorption after application.

On the other hand, as shown in Table 3, none of the emulsion cosmetics of Comparative Examples 6 to 13 were evaluated good in all evaluation items.

That is, the emulsion cosmetic of Comparative Example 9 containing the oil agent for cosmetics of Comparative Example 1 not containing component (B) was evaluated as having an insufficient feeling of thickness when applied to the skin in a high temperature and high humidity state, a bad feeling of penetration into the skin in a high temperature and high humidity state, and an unpleasant sense of adsorption after application.

The emulsion cosmetic of Comparative Example 10 containing the oil agent for cosmetics of Comparative Example 2 not containing component (A) was evaluated as having a bad feeling of thickness when applied to the skin in a high temperature and high humidity state, an insufficient feeling of penetration into the skin in a high temperature and high humidity state, and an unpleasant sense of adsorption after application.

The emulsion cosmetic of Comparative Example 11 containing the oil agent for cosmetics of Comparative Example 3, in which the content of component (A) is less than 70% by mass and the content of component (B) exceeds 30% by mass, was evaluated as having an insufficient feeling of thickness when applied to the skin in a high temperature and high humidity state and slightly unpleasant feeling after application.

The emulsion cosmetic of Comparative Example 12 containing the oil agent for cosmetics of Comparative Example 4, in which a part of component (A) was replaced with component (A') (linear primary alcohol having 22 carbon atoms) and the content of component (A) was less than 70% by mass, was evaluated as having a bad feeling of penetration into the skin in a high temperature and high humidity state, and an unpleasant sense of adsorption after application.

The emulsion cosmetic of Comparative Example 13 containing the oil agent for cosmetics of Comparative Example 5, which contained component (B') (linear primary alcohol having 16 carbon atoms) instead of component (B), was evaluated as having a bad feeling of penetration into the skin in a high temperature and high humidity state, and a slightly unpleasant sense of adsorption after application.

The emulsion cosmetic of Comparative Example 6 containing the oil agent for cosmetics of Example 5 at a content of less than 1% by mass, and the emulsion cosmetic of Comparative Example 8 containing the oil agent for cosmetics of Example 5, in which the content exceeds 60% by mass were evaluated as having an insufficient feeling of penetration into the skin in a high temperature and high humidity state, and a slightly unpleasant sense of adsorption after application.

The emulsion cosmetic of Comparative Example 7 not containing component (C) was evaluated as having a bad feeling of penetration into the skin in a high temperature and high humidity state, and an unpleasant sense of adsorption after application.

Further examples of the oil agent for cosmetic of the present invention and the the emulsion cosmetic of the present invention are shown.

### [Example 18] oil agent for cosmetics

The oil agent for cosmetics of Example 18 was prepared by uniformly mixing the components in Table 5 according to the composition shown in Table 5. As the component (A) and component (B) in Table 5, commercially available products provided for use in cosmetics were used.

The oil agent for cosmetics of Example 18 was evaluated for the feeling of thickness when applied to the skin in a high temperature and high humidity state and the feeling of penetration into the skin in a high temperature and high humidity state, as in the case of the oil agents for cosmetics of the above-mentioned Examples 1 to 7 and Comparative Examples 1 to 5, and the evaluation results are also shown in Table 5.

**[Table 5]**

| component | | content (% by mass) |
|---|---|---|
| (A) | 2-octyl-1-dodecanol | 97 |
| (B) | 2-pentyl-1-dodecanol | 3 |
| total | | 100 |
| evaluation item | feeling of thickness when applied to skin in high temperature and high humidity state | ⊙ (39) |
| | feeling of penetration into skin in high temperature and high humidity state | ○ (34) |

| | | |
|---|---|---|
| *; The numerical value in parenthesis in the evaluation results indicates the total score. | | |

As shown in Table 5, oil agent for cosmetics of Example 18 was evaluated as an oil agent for cosmetics having a very good feeling of thickness when applied to the skin in a high temperature and high humidity state, and also a good feeling of penetration into the skin in a high temperature and high humidity state.

### [Example 19] emulsion cosmetics

According to the compositions shown in Table 6, the oil agent for cosmetics of Example 18 (component (A) and component (B)), component (C), component (D), and component (E) shown in Table 6, the common addition component shown in Table 4, and water were mixed, heated to 75°C to 80°C, a shear force was applied using a homomixer to mix and emulsify at that temperature, and then the mixture was cooled to prepare the emulsion cosmetic of Example 19. As component (C), component (D), and component (E) in Table 6 and each component shown in Table 4, commercially available products provided for use in cosmetics were used.

The emulsion cosmetic of Example 19 was evaluated for (1) a feeling of thickness when applied to the skin in a high temperature and high humidity state, (2) a feeling of penetration into the skin in a high temperature and high humidity state, and (3) an unpleasant sense of adsorption after application, as in the case of Examples 8 to 17 and Comparative Examples 6 to 13, and the evaluation results are also shown in Table 6.

**[Table 6]**

| component | | content (% by mass) |
|---|---|---|
| (A) and (B) | oil agent for cosmetics of Example 18 | 5.0 |
| (C) | 1,3-butylene glycol | 10.0 |
| | dipropylene glycol | 5.0 |
| (D) | decaglyceryl monostearate (HLB=14) | 4.0 |
| (E) | olive oil | 2.5 |
| | sunflower oil | 2.5 |
| common addition component | | 4.0 |
| water | | rest |
| total | | 100.0 |
| (A+B)/C | | 0.3 |
| evaluation item | feeling of thickness when applied to skin in high temperature and high humidity state | ⊙ (39) |
| | feeling of penetration into skin in high temperature and high humidity state | ○ (34) |
| | unpleasant sense of adsorption after application | ○ (34) |

| | | |
|---|---|---|
| *; The numerical value in parenthesis in the evaluation results indicates the total score. | | |

As shown in Table 6, the emulsion cosmetic of Examples 19 containing the oil agent for cosmetics of Example 18 was evaluated to be an emulsion cosmetic having a very good feeling of thickness when applied to the skin in a high temperature and high humidity state and a good feeling of penetration into the skin in a high temperature and high humidity state, with less unpleasant sense of adsorption after application.

Successively, formulation examples (Formulation Examples 1 - 4) of cosmetics containing the oil agent for cosmetic of the present invention are shown.

### [Formulation Example 1] hair oil

According to the compositions shown in Table 7, the oil agent for cosmetics of Example 4 (component (A) and component (B)), oil agent other than component (A) and component (B), hair conditioning agent, antioxidant and flavor in Table 7 are mixed and uniformly dissolved to prepare hair oil. As the oil agent other than component (A) and component (B), hair conditioning agent, antioxidant and flavor in Table 7, commercially available products provided for use in cosmetics are used.

The hair oil of Formulation Example 1 has both a good feeling of thickness when applied to the skin and hair in a high temperature and high humidity state, and a good feeling of penetration into the skin and hair in a high temperature and high humidity state.

**[Table 7]**

| component | | content (% by mass) |
|---|---|---|
| (A) and (B) | oil agent for cosmetics of Example 4 | 25 |
| oil agent other than (A) and (B) | olive oil | 10 |
| | dimethylpolysiloxane | rest |
| hair conditioning agent | methylsiloxane-methyl(aminopropyl)siloxane copolymer | 5 |
| antioxidant | tocopherol | 0.05 |
| flavor | | 0.3 |
| total | | 100 |

### [Formulation Example 2] point makeup remover

According to the compositions shown in Table 8, the oil agent for cosmetics of Example 18 (component (A) and component (B)), oil agent other than component (A) and component (B), and antioxidant in Table 8 are uniformly mixed and layered on an aqueous layer prepared by dissolving lower alcohol, a humectant and a stabilizer in water to prepare a point makeup remover. As the oil agent other than component (A) and component (B), antioxidant, lower alcohol, humectant and stabilizer in Table 8, commercially available products provided for use in cosmetics are used.

The point makeup remover of Formulation Example 2 has both a good feeling of thickness when applied to the skin in a high temperature and high humidity state, and a good feeling of penetration into the skin in a high temperature and high humidity state.

**[Table 8]**

| component | | content (% by mass) |
|---|---|---|
| (A) and (B) | oil agent for cosmetics of Example 18 | 25 |
| oil agent other than (A) and (B) | tri(caprylic acid/capric acid) glyceride | 1.5 |
| | olive oil | 1 |
| | cetyl ethylhexanoate | 15 |
| antioxidant | tocopherol | 0.05 |
| lower alcohol | ethanol | 5 |
| humectant | dipropylene glycol | 7 |
| | glycerol | 3 |
| | pentylene glycol | 1 |
| | 1,2-hexanediol | 0.3 |
| | 1,3-butylene glycol | 1 |
| stabilizer | sodium chloride | 1 |
| | disodium ethylenediaminetetraacetate | 0.25 |
| water | | rest |
| total | | 100 |

### [Formulation Example 3] cleansing oil

According to the compositions shown in Table 9, the oil agent for cosmetics of Example 4 (component (A) and component (B)), oil agent other than component (A) and component (B), antioxidant and lipophilic surfactant in Table 9 are mixed, a hydrophilic surfactant and a flavor are mixed, dissolved and added to prepare cleansing oil. As the oil agent other than component (A) and component (B), antioxidant, lipophilic surfactant, hydrophilic surfactant, and flavor in Table 9, commercially available products provided for use in cosmetics are used.

The cleansing oil of Formulation Example 3 has both a good feeling of thickness when applied to the skin in a high temperature and high humidity state, and a good feeling of penetration into the skin in a high temperature and high humidity state.

**[Table 9]**

| component | | content (% by mass) |
|---|---|---|
| (A) and (B) | oil agent for cosmetics of Example 4 | 20 |
| oil agent other than (A) and (B) | olive oil | 10 |
| | cetyl ethylhexanoate | rest |
| | squalane | 5 |
| | ethyl oleate | 6 |
| antioxidant | tocopherol | 0.05 |
| lipophilic surfactant | sorbitan oleate | 4 |
| | glyceryl laurate | 3 |
| hydrophilic surfactant | polyoxyethylene (30E.O.) sorbitol tetraoleate | 15 |
| | decaglyceryl diisostearate | 6 |
| | polyoxyethylene (20E.O.) sorbitan monolaurate | 7 |
| flavor | | 0.3 |
| total | | 100 |

### [Formulation Example 4] hair treatment

According to the compositions shown in Table 10, the oil agent for cosmetics of Example 4 (component (A) and component (B)), an oil agent other than component (A) and component (B), hair conditioning agent, antioxidant, surfactant, humectant, pH adjuster, antiseptic and flavor in Table 10 are added to water, and a shear force is applied to mix them to prepare a hair treatment. As the oil agent other than component (A) and component (B), hair conditioning agent, antioxidant, surfactant, humectant, pH adjuster, antiseptic and flavor, commercially available products provided for use in cosmetics are used.

The hair treatment of Formulation Example 4 shows both a good feeling of thickness when applied to the skin and hair in a high temperature and high humidity state, and a good feeling of penetration into the skin and hair in a high temperature and high humidity state.

**[Table 10]**

| component | | content (% by mass) |
|---|---|---|
| (A) and (B) | oil agent for cosmetics of Example 4 | 5 |
| oil agent other than (A) and (B) | squalane | 1 |
| | behenyl alcohol | 1 |
| | cetanol | 4 |
| | dimethylpolysiloxane | 5 |
| hair conditioning agent | methylsiloxane-methyl(aminopropyl)siloxane copolymer | 0.5 |
| | aminoethylaminopropylmethylsiloxane-dimethylsiloxane copolymer | 0.5 |
| antioxidant | tocopherol | 0.05 |
| surfactant | behenyl trimethylammonium chloride | 1.5 |
| | stearic acid dimethylaminopropylamide | 0.5 |
| humectant | glycerol | 5 |
| pH adjuster | citric acid | appropriate amount |
| | sodium citrate | appropriate amount |
| antiseptic | phenoxyethanol | 5 |
| flavor | | 0.3 |
| water | | rest |
| total | | 100 |

Successively, formulation examples (Formulation Examples 5, 6) of the emulsion cosmetic of the present invention are shown.

### [Formulation Example 5] milky lotion

According to the compositions shown in Table 11, the oil agent for cosmetics of Example 4 (component (A) and component (B)), component (E), lipophilic additive, component (C), component (D), hydrophilic additive, and water in Table 11 are mixed, heated to 75°C to 80°C, a shear force is applied using a homomixer to mix and emulsify, and then the mixture is cooled to prepare milky lotion. As component (E), lipophilic additive, component (C), component (D), and hydrophilic additive, commercially available products provided for use in cosmetics are used.

The milky lotion of Formulation Example 5 shows both a good feeling of thickness when applied to the skin in a high temperature and high humidity state, and a good feeling of penetration into the skin in a high temperature and high humidity state, and shows less unpleasant sense of adsorption after application.

**[Table 11]**

| component | | content (% by mass) |
|---|---|---|
| (A) and (B) | oil agent for cosmetics of Example 4 | 5 |
| (E) | olive oil | 2 |
| lipophilic additive | squalane | 2 |
| | dimethylpolysiloxane | 1 |
| | ethylhexyl palmitate | 2 |
| | glycerol mono 2-ethylhexylether | 0.1 |
| (C) | glycerol | 5 |
| | 1,3-butylene glycol | 2 |
| (D) | pentaglyceryl monostearate (HLB value = 12.9) | 3 |
| hydrophilic additive | PEG/PPG/polybutyleneglycol-8/5/3 glycerol (addition copolymer of ethylene oxide, propylene oxide and butylene oxide (8 mol, 5 mol and 3 mol) to glycerol) | 0.5 |
| | 2-methacryloyloxyethylphosphoryl choline-butyl methacrylate copolymer solution | 0.1 |
| | sodium hyaluronate (1% by mass aqueous solution) | 0.01 |
| | dipotassium glycyrrhizate | 1 |
| | phenoxyethanol | 0.3 |
| | carboxyvinyl polymer | 0.3 |
| | potassium hydroxide | appropriate amount |
| water | | rest |
| total | | 100 |

### [Formulation Example 6] cream

According to the compositions shown in Table 12, the oil agent for cosmetics of Example 18 (component (A) and component (B)), component (E), lipophilic additive, component (C), component (D), hydrophilic additive, and water in Table 12 are mixed, heated to 75°C to 80°C, a shear force is applied using a homomixer to mix and emulsify, and the mixture is cooled to prepare a cream. As the component (E), lipophilic additive, component (C), component (D), and hydrophilic additive, commercially available products provided for use in cosmetics are used.

The cream of Formulation Example 6 shows both a good feeling of thickness when applied to the skin in a high temperature and high humidity state, and a good feeling of penetration into the skin in a high temperature and high humidity state, with less unpleasant sense of adsorption after application.

**[Table 12]**

| component | | content (% by mass) |
|---|---|---|
| (A) and (B) | oil agent for cosmetics of Example 18 | 7.5 |
| (E) | olive oil | 2 |
| lipophilic additive | squalane | 2 |
| | ethylhexyl palmitate | 2 |
| | cetanol | 2 |
| | behenyl alcohol | 2 |
| | beeswax | 1 |
| | tocopherol | 0.05 |
| | glycerol mono 2-ethylhexylether | 0.1 |
| (C) | glycerol | 5 |
| | propylene glycol | 3 |
| | 1,3-butylene glycol | 2 |
| (D) | pentaglyceryl monolaurate (HLB value=15.8) | 3 |
| hydrophilic additive | PEG/PPG/polybutyleneglycol-8/5/3 glycerol (addition copolymer of ethylene oxide, propylene oxide and butylene oxide (8 mol, 5 mol and 3 mol) to glycerol) | 0.5 |
| | 2-methacryloyloxyethylphosphoryl choline-butyl methacrylate copolymer solution | 0.1 |
| | sodium hyaluronate (1% by mass aqueous solution) | 0.01 |
| | niacin amide | 3 |
| | phenoxyethanol | 0.2 |
| | citric acid | appropriate amount |
| | sodium citrate | appropriate amount |
| water | | rest |
| total | | 100 |

Next, examples of the powder-containing cosmetic of the present invention are shown.

### [Examples 20 - 29, Comparative Examples 14 - 18] powder-containing oil-in-water emulsion cosmetics

According to the compositions shown in Table 13, the component (A), component (B), component (F), lipophilic additive, common addition component and purified water in Table 13 were mixed, heated to 75°C to 80°C, a shear force was applied using a homomixer to mix and emulsify at that temperature, and then the mixture was cooled to prepare the powder-containing oil-in-water emulsion cosmetics of Examples 20 to 29.

According to the compositions shown in Table 14, the component (A), component (A'), component (B) or component (B'), component (F), common addition component, and purified water in Table 14 were mixed, heated to 75°C to 80°C, a shear force was applied using a homomixer to mix and emulsify at that temperature, and then the mixture was cooled to prepare the powder-containing oil-in-water emulsion cosmetics of Comparative Examples 14 to 18.

The common addition components in Tables 13, 14 are shown in Table 15. As the component (A), component (A'), component (B), component (B'), component (F), and lipophilic additive in Tables 13, 14, and each component shown in Table 15, commercially available products provided for use in cosmetics were used.

Each of the powder-containing oil-in-water emulsion cosmetics of Examples 20 to 29 and Comparative Examples 14 to 18 was evaluated for a feeling of dryness after application to the skin, a sense of friction when applied to the skin, a feeling of thickness when applied to the wet skin, and a feeling of blending when applied to the wet skin, as shown below. The evaluation results are also shown in Tables 13, 14.

### (1) feeling of dryness

20 female panelists (22 years old to 40 years old) applied about 0.1 g of each of the powder-containing oil-in-water emulsion cosmetics of Examples and Comparative Examples to an inner part of the forearm, and evaluated the feeling of dryness that was felt after blending with fingers, according to the following evaluation criteria. The total score of the 20 panelists was calculated, and a feeling of dryness after application to the skin was determined based on the total score and according to the following determination criteria. A powder-containing oil-in-water emulsion cosmetic that received a rating of "○" or "⊙" was evaluated as a powder-containing oil-in-water emulsion cosmetic that does not easily cause a feeling of dryness after application to the skin.

### <Evaluation criteria>

2 points: feeling of dryness was not felt
1 point: slight feeling of dryness was felt
0 point: feeling of dryness was clearly felt

### <Determination criteria>

⊙: the total score is 35 points or more
O: the total score is 30 points or more and 34 points or less
Δ: the total score is 20 points or more and 29 points or less
×: the total score is 19 points or less

### (2) sense of friction

20 female panelists (22 years old to 40 years old) applied about 0.1 g of each of the powder-containing oil-in-water emulsion cosmetics of Examples and Comparative Examples to an inner part of the forearm, and evaluated the feeling of friction that was felt when blending with fingers, according to the following evaluation criteria. The total score of the 20 panelists was calculated, and a feeling of friction when applied to the skin was determined based on the total score and according to the following determination criteria. A powder-containing oil-in-water emulsion cosmetic that received a rating of "○" or "⊙" was evaluated as a powder-containing oil-in-water emulsion cosmetic that does not easily cause a feeling of friction when applied to the skin.

### <Evaluation criteria>

2 points: feeling of friction was not felt
1 point: slight feeling of friction was felt
0 point: feeling of friction was clearly felt

### <Determination criteria>

⊙: the total score is 35 points or more
O: the total score is 30 points or more and 34 points or less
Δ: the total score is 20 points or more and 29 points or less
×: the total score is 19 points or less

### (3) feeling of thickness when applied to the wet skin

20 female panelists (22 years old to 40 years old) sprayed one push (0.4 mL) of tap water at room temperature onto the forearm and then blended in with the fingers for 10 seconds. Immediately thereafter, they applied about 0.1 g of each of the powder-containing oil-in-water emulsion cosmetics of Examples and Comparative Examples to an inner part of the forearm, and evaluated the feeling of thickness that was felt when blending with fingers, according to the following evaluation criteria. The total score of the 20 panelists was calculated, and a feeling of thickness that was felt when applied to the wet skin was determined based on the total score and according to the following determination criteria. A powder-containing oil-in-water emulsion cosmetic that received a rating of "○" or "⊙" was evaluated as a powder-containing oil-in-water emulsion cosmetic with a good feeling of thickness when applied to the wet skin.

### <Evaluation criteria>

2 points: when applied to the skin, very good feeling of thickness was felt
1 point: when applied to the skin, slightly good feeling of thickness was felt
0 point: when applied to the skin, slightly bad feeling of thickness was felt

### <Determination criteria>

⊙: the total score is 35 points or more
○: the total score is 30 points or more and 34 points or less
Δ: the total score is 20 points or more and 29 points or less
×: the total score is 19 points or less

### (4) feeling of blending when applied to the wet skin

20 female panelists (22 years old to 40 years old) sprayed one push (0.4 mL) of tap water at room temperature onto the forearm and then blended in with the fingers for 10 seconds. Immediately thereafter, they applied about 0.1 g of each of the powder-containing oil-in-water emulsion cosmetics of Examples and Comparative Examples to an inner part of the forearm, and evaluated the feeling of blending that was felt when blending with fingers, according to the following evaluation criteria. The total score of the 20 panelists was calculated, and a feeling of blending when applied to the wet skin was determined based on the total score and according to the following determination criteria. A powder-containing oil-in-water emulsion cosmetic that received a rating of "○" or "⊙" was determined as a powder-containing oil-in-water emulsion cosmetic with a good feeling of blending when applied to the wet skin.

### <Evaluation criteria>

2 points: when applied to the skin, very good feeling of blending into the skin was felt
1 point: when applied to the skin, slightly good feeling of blending into the skin was felt
0 point: when applied to the skin, slightly bad feeling of blending into the skin was felt

### <Determination criteria>

⊙: the total score is 35 points or more
○: the total score is 30 points or more and 34 points or less
Δ: the total score is 20 points or more and 29 points or less
×: the total score is 19 points or less

**[Table 13]**

| component | | Example | | | | | | | | | |
|---|---|---|---|---|---|---|---|---|---|---|---|
| | | 20 | 21 | 22 | 23 | 24 | 25 | 26 | 27 | 28 | 29 |
| (A) | 2-octyl-1-dodecanol | 16.0 | | | 6.0 | 2.9 | 4.5 | 8.0 | 4.5 | 33.0 | 18.0 |
| | 2-decyl-1-tetradecanol | | 12.0 | 28.0 | | | | | | | |
| | 2-dodecyl-1-hexadecanol | | | | 12.0 | | | | | | |
| (B) | 2-pentyl-1-nonanol | 4.0 | | | | | | | | 11.0 | 6.0 |
| | 2-pentyl-1-dodecanol | | | | | 0.2 | 0.5 | | | | |
| | 2-octyl-1-decanol | | 3.0 | 7.0 | 5.0 | | | 2.0 | | | |
| | 16-methyl-1-heptadecanol | | | | | | | | 0.5 | | |
| (F) | particulate titanium oxide (average particle size=10 nm) | 1.0 | 12.5 | 0.5 | 5.0 | 10.0 | 10.0 | 10.0 | 15.0 | 7.0 | 12.5 |
| | particulate zinc oxide (average particle size=30 nm) | 1.0 | 12.5 | 0.5 | 5.0 | 10.0 | 10.0 | 10.0 | 15.0 | 7.0 | 12.5 |
| | titanium oxide (average particle size=200 nm) | | | 3.0 | 2.0 | | | | 2.0 | 1.0 | |
| | iron oxide (average particle size=500 nm) | | | 0.05 | | | | | | | 0.05 |
| | silica (average particle size=5 µm) | | | 1.0 | | | | 2.0 | | | |
| | talc (average particle size=20 µm) | | 1.0 | 2.0 | 1.0 | | | | 2.0 | | |
| | mica (average particle size=100 µm) | | 2.0 | 2.0 | | | | | | | |
| lipophilic additive | 2-ethylhexyl paramethoxycinnamate | 5.0 | | 2.0 | | 7.0 | 7.0 | | 3.0 | | |
| | hexyl 2-[4-(diethylamino)-2-hvdroxvbenzovl]benzoate | 1.0 | | | | 2.0 | 2.0 | | 1.0 | | |
| | 4-t-butyl-4'-methoxydibenzoylmethane | 1.0 | | | | 2.0 | 2.0 | | 1.0 | | |
| common addition component | | 10.1 | | | | | | | | | |
| purified water | | rest | | | | | | | | | |
| total | | 100 | | | | | | | | | |
| (A) / (B) | | 4.0 | 4.0 | 4.0 | 3.6 | 14.5 | 9.0 | 4.0 | 9.0 | 3.0 | 3.0 |
| total amount of component (F) | | 2.0 | 28.0 | 9.05 | 13.0 | 20.0 | 20.0 | 22.0 | 34.0 | 15.0 | 25.05 |
| (A)+(B) | | 20.0 | 15.0 | 35.0 | 23.0 | 3.1 | 5.0 | 10.0 | 5.0 | 44.0 | 24.0 |
| evaluation item | feeling of dryness | ○ (33) | ⊙ (35) | ○ (33) | ⊙ (35) | ⊙ (35) | ⊙ (35) | ⊙ (35) | ⊙ (37) | ○ (34) | ○ (32) |
| | sense of friction | ⊙ (35) | ○ (31) | ○ (30) | ○ (30) | ⊙ (35) | ⊙ (36) | ⊙ (35) | ⊙ (36) | ○ (32) | ⊙ (35) |
| | feeling of thickness when applied to wet skin | ○ (30) | ⊙ (35) | ○ (31) | ○ (31) | ⊙ (37) | ⊙ (38) | ⊙ (38) | ⊙ (36) | ○ (30) | ○ (32) |
| | feeling of blending when applied to wet skin | ⊙ (38) | ○ (33) | ○ (31) | ○ (30) | ○ (33) | ⊙ (35) | ⊙ (37) | ⊙ (35) | ○ (31) | ○ (34) |

| | | | | | | | | | | | |
|---|---|---|---|---|---|---|---|---|---|---|---|
| *; The numerical value for each component in the table indicates the content (% by mass) of the component, and a blank column indicates that the component is not contained. | | | | | | | | | | | |

**[Table 14]**

| component | | Comparative Example | | | | |
|---|---|---|---|---|---|---|
| | | 14 | 15 | 16 | 17 | 18 |
| (A) | 2-octyl-1-dodecanol | 5.0 | | 2.0 | 8.0 | 8.0 |
| | 2-decyl-1-tetradecanol | | | | | |
| | 2-dodecyl-1-hexadecanol | | | | | |
| (A') | behenyl alcohol | | | 2.0 | | |
| (B) | 2-pentyl-1-nonanol | | | | | |
| | 2-pentyl-1-dodecanol | | | | | |
| | 2-octyl-1-decanol | | 5.0 | 1.0 | | 2.0 |
| | 16-methyl-1-heptadecanol | | | | | |
| (B') | cetanol | | | | 2.0 | |
| (F) | particulate titanium oxide (average particle size=10 nm) | 16.0 | 15.0 | 10.0 | 10.0 | 0.5 |
| | particulate zinc oxide (average particle size=30 nm) | 16.0 | 15.0 | 10.0 | 10.0 | 0.5 |
| | titanium oxide (average particle size=200 nm) | 2.0 | | | | |
| | iron oxide (average particle size=500 nm) | | | | | |
| | silica (average particle size=5 µm) | 2.0 | | | 2.0 | |
| | talc (average particle size=20 µm) | | | 2.0 | | |
| | mica (average particle size=100 µm) | | 2.0 | | | |
| common addition component | | 10.1 | | | | |
| purified water | | rest | | | | |
| total | | 100 | | | | |
| (A) / (B) | | - | 0.0 | 2.0 | - | 4.0 |
| total amount of component (F) | | 36.0 | 32.0 | 22.0 | 22.0 | 1.0 |
| (A)+(B) | | 5.0 | 5.0 | 3.0 | 8.0 | 10.0 |
| evaluation item | feeling of dryness | Δ (23) | × (19) | Δ (22) | Δ (24) | ⊙ (38) |
| | sense of friction | Δ (22) | Δ (25) | Δ (26) | Δ (21) | ⊙ (37) |
| | feeling of thickness when applied to wet skin | Δ (29) | Δ (27) | Δ (28) | ○ (31) | Δ (25) |
| | feeling of blending when applied to wet skin | × (19) | Δ (23) | Δ (24) | Δ (20) | Δ (24) |

| | | | | | | |
|---|---|---|---|---|---|---|
| *; The numerical value for each component in the table indicates the content (% by mass) of the component, and a blank column indicates that the component is not contained. **; In the table, "-" indicates that the numerical value cannot be calculated. | | | | | | |

**[Table 15]**

| component | content (% by mass) |
|---|---|
| alkyl acrylate copolymer | 0.5 |
| xanthan gum | 0.3 |
| beeswax | 4 |
| glyceryl monostearate | 1 |
| decaglyceryl monolaurate | 2 |
| decaglyceryl monostearate | 2 |
| phenoxyethanol | 0.3 |
| total | 10.1 |

As shown in Table 13, the powder-containing oil-in-water emulsion cosmetics of Example 20 to 29 were all evaluated as powder-containing oil-in-water emulsion cosmetics that do not easily cause a feeling of dryness after application or a sense of friction when applied, and have a good feeling of thickness when applied to the wet skin and a good feeling of blending when applied to the wet skin.

In particular, the powder-containing oil-in-water emulsion cosmetics of Examples 25 to 27 containing 2-octyl-1-dodecanol as component (A), containing 2-pentyl-1-dodecanol, 2-octyl-1-decanol, or 16-methyl-1-heptadecanol as component (B), and having a (A)/(B) value of 4 or more and 10 or less, in which the total content of components (A) and (B) is 3% by mass or more and 20% by mass or less, the total content of component (F) is 18% by mass or more and 35% by mass or less, were determined as **"⊙"** in all evaluation items, and evaluated to be extremely unlikely to cause a feeling of dryness after application and a sense of friction when applied, and have a very good feeling of thickness when applied to the wet skin and a very good feeling of blending when applied to the wet skin.

On the other hand, as shown in Table 14, none of the powder-containing oil-in-water emulsion cosmetics of Comparative Examples 14 to 18 received an evaluation that they were good powder-containing oil-in-water emulsion cosmetics in all evaluation items.

That is, in the powder-containing oil-in-water emulsion cosmetic of Comparative Example 14 not containing component (B), a feeling of dryness after application and a sense of friction when applied were slightly felt, and it was evaluated as having an insufficient feeling of thickness when applied to the wet skin and a bad feeling of blending when applied to the wet skin.

The powder-containing oil-in-water emulsion cosmetic of Comparative Example 15 not containing component (A) was evaluated as having a feeling of dryness after application and a slight sense of friction when applied. In addition, it was evaluated as having an insufficient feeling of thickness when applied to the wet skin and an insufficient feeling of blending when applied to the wet skin.

The powder-containing oil-in-water emulsion cosmetic of Comparative Example 16 containing a linear primary alcohol having 22 carbon atoms as component (A') along with component (A) and having an (A)/(B) value of less than 2.3 was evaluated as having a slight feeling of dryness after application and a slight sense of friction when applied, as well as having an insufficient feeling of thickness when applied to the wet skin and an insufficient feeling of blending when applied to the wet skin.

The powder-containing oil-in-water emulsion cosmetic of Comparative Example 17 containing a linear primary alcohol having 16 carbon atoms as component (B') instead of component (B) was evaluated as having a slight feeling of dryness after application and a slight sense of friction when applied, and having an insufficient feeling of blending when applied to the wet skin.

The powder-containing oil-in-water emulsion cosmetic of Comparative Example 18 with a content of component (F) of less than 2% by mass was evaluated as having an insufficient feeling of thickness when applied to the wet skin and an insufficient feeling of blending when applied to the wet skin.

Successively, examples of the powder-containing water-in-oil emulsion cosmetics of the present invention are shown.

### [Example 30] powder-containing water-in-oil type emulsion cosmetic

According to the compositions shown in Table 16, component (A), component (B), oil agent other than component (A) and component (B), surfactant, and lipophilic additive in the Table were mixed, component (F) was added and dispersed therein, and the mixture was heated to 75°C to 80°C to prepare an oil phase, an aqueous phase prepared by adding a hydrophilic additive to purified water, mixing, dissolving, and heating same to 75°C to 80°C was added thereto, and the mixture was uniformly mixed and emulsified using a homomixer to obtain the powder-containing water-in-oil emulsion cosmetic of Example 30.

As the components (A), (B), and (F), oil agent other than component (A) and component (B), surfactant, lipophilic additive, and hydrophilic additive in Table 16, commercially available products provided for use in cosmetics were used.

The powder-containing water-in-oil emulsion cosmetic of Example 30 is preferably used as a sunscreen cream.

**[Table 16]**

| component | | content (% by mass) |
|---|---|---|
| (A) | 2-octyl-1-dodecanol | 18 |
| (B) | 16-methyl-1-heptadecanol | 2 |
| oil agent other than (A) and (B) | isopropyl myristate | 1 |
| | dimethylpolysiloxane | 2 |
| lipophilic additive | 2-ethylhexyl paramethoxycinnamate | 7 |
| | hexyl 2-[4-(diethylamino)-2-hydroxybenzoyl]benzoate | 2 |
| | 4-t-butyl-4'-methoxydibenzoylmethane | 2 |
| (F) | particulate titanium oxide (average particle size=10 nm) | 5 |
| | particulate zinc oxide (average particle size=30 nm) | 5 |
| | talc (average particle size=20 µm) | 1 |
| | silica (average particle size=5 µm) | 1 |
| surfactant | polyoxyethylene-methylpolysiloxane copolymer | 3 |
| hydrophilic additive | glycerol | 2 |
| | 1,3-butylene glycol | 3 |
| | glycerol mono 2-ethylhexylether | 0.1 |
| | phenoxyethanol | 0.2 |
| purified water | | rest |
| total | | 100 |

### [Example 31] powder-containing water-in-oil emulsion cosmetic

According to the compositions shown in Table 17, component (A), component (B), oil agent other than component (A) and component (B), surfactant, and lipophilic additive in the Table were mixed, component (F) was added and dispersed therein, and the mixture was heated to 75°C to 80°C to prepare an oil phase, an aqueous phase prepared by adding a hydrophilic additive to purified water, mixing, dissolving, and heating same to 75°C to 80°C was added thereto, and the mixture was uniformly mixed and emulsified using a homomixer to obtain the powder-containing water-in-oil emulsion cosmetic of Example 31.

As the components (A), (B), and (F), oil agent other than component (A) and component (B), surfactant, lipophilic additive, and hydrophilic additive in Table 17, commercially available products provided for use in cosmetics were used.

The powder-containing water-in-oil emulsion cosmetic of Example 31 is preferably used as a creamy foundation.

**[Table 17]**

| component | | content (% by mass) |
|---|---|---|
| (A) | 2-octyl-1-dodecanol | 12 |
| (B) | 2-pentyl-1-dodecanol | 3 |
| oil agent other than (A) and (B) | dodecane | 10 |
| | dimethylpolysiloxane | 2 |
| lipophilic additive | 2-ethylhexyl paramethoxycinnamate | 5 |
| | 2-cyano-3,3-diphenyl-2-propenoic acid 2-ethylhexyl(octocrylene) | 2.5 |
| (F) | particulate titanium oxide (average particle size=10 nm) | 2.5 |
| | particulate zinc oxide (average particle size=30 nm) | 2.5 |
| | titanium oxide (average particle size=200 nm) | 5 |
| | talc (average particle size=20 µm) | 5 |
| | silica (average particle size=5 µm) | 5 |
| | iron oxide (average particle size=500 nm) | 2.5 |
| surfactant | diglyceryl monostearate | 2 |
| | polyoxyethylene-methylpolysiloxane copolymer | 3 |
| hydrophilic additive | glycerol | 3 |
| | glycerol mono 2-ethylhexylether | 0.1 |
| | phenoxyethanol | 0.2 |
| purified water | | rest |
| total | | 100 |

Successively, formulation examples (Formulation Examples 7, 8) of the powder-containing cosmetic of the present invention are shown.

### [Formulation Example 7] lip rouge

According to the compositions shown in Table 18, the oil agent for cosmetics of Example 18 (component (A) and component (B)), oil agent other than component (A) and component (B), and lipophilic additive in Table 18 are mixed, uniformly melt, and a dye and component (F) mixed with a surface modifier are added and dispersed uniformly, and the mixture is filled into a mold and shaped to prepare a lip rouge. As the oil agent other than component (A) and component (B), lipophilic additive, surface modifier, dye and component (F), commercially available products provided for use in cosmetics are used.

The lip rouge of Formulation Example 7 suppresses a feeling of dryness and a sense of friction due to the inclusion of inorganic powder, and can simultaneously achieve a feeling of thickness and a feeling of blending into lips when applied to wet lips.

**[Table 18]**

| component | | content (% by mass) |
|---|---|---|
| (A) and (B) | oil agent for cosmetics of Example 18 | 7 |
| oil agent other than (A) and (B) | castor oil | 5 |
| | olive oil | 5 |
| | jojoba oil | 5 |
| | Carnauba wax | 2 |
| | polyethylene wax | 9 |
| | dimethylpolysiloxane (SH100) | 5 |
| | hydrogenated polyisobutene | rest |
| lipophilic additive | silicone resin | 18 |
| surface modifier | aluminum hydroxide | appropriate amount |
| dye | Red 201 | 1 |
| (F) | pearl pigment | 5 |
| total | | 100 |

### [Formulation Example 8] liquid foundation

According to the compositions shown in Table 19, the oil agent for cosmetics of Example 4 (component (A) and component (B)), oil agent other than component (A) and component (B), lipophilic additive, surfactant, hydrophilic additive, and purified water in Table 19 are mixed, heated to 75°C to 80°C, component (F) mixed with a surface modifier is added and uniformly dispersed, and the mixture is mixed and emulsified using a homomixer to prepare a liquid foundation. As the oil agent other than component (A) and component (B), lipophilic additive, surfactant, hydrophilic additive, surface modifier, and component (F), commercially available products provided for use in cosmetics are used.

The liquid foundation of Formulation Example 8 suppresses a feeling of dryness and a sense of friction due to the inclusion of inorganic powder, and can simultaneously achieve a feeling of thickness and a feeling of blending into skin when applied to the wet skin.

**[Table 19]**

| component | | content (% by mass) |
|---|---|---|
| (A) and (B) | oil agent for cosmetics of Example 4 | 10 |
| oil agent other than (A) and (B) | dimethylpolysiloxane | 3 |
| | beeswax | 1 |
| | hydrogenated polyisobutene | 1 |
| | stearic acid | appropriate amount |
| lipophilic additive | tocopherol | 0.05 |
| | glycerol mono 2-ethylhexylether | 0.1 |
| surfactant | polyoxyethylene-methylpolysiloxane copolymer | 2 |
| | 1,3-butylene glycol | 5 |
| | dipropylene glycol | 5 |
| | propanediol | 3 |
| | PEG/PPG/polybutyleneglycol-8/5/3 glycerol (addition copolymer of ethylene oxide, propylene oxide and butylene oxide (8 mol, 5 mol and 3 mol) to glycerol) | 0.5 |
| | 2-methacryloyloxyethylphosphoryl choline-butyl methacrylate copolymer solution | 0.01 |
| hydrophilic additive | sodium hyaluronate (1% by mass aqueous solution) | 0.01 |
| | dipotassium glycyrrhizate | 1 |
| | phenoxyethanol | 0.5 |
| | (acrylates/alkyl(C10-30) acrylate) crosslinked polymer | 0.2 |
| | carboxyvinyl polymer | 0.1 |
| | xanthan gum | 0.3 |
| | arginine | appropriate amount |
| surface modifier | aluminum hydroxide | appropriate amount |
| (F) | silica | 1 |
| | talc | 3 |
| | titanium oxide | 8 |
| | iron oxide | 2 |
| purified water | | rest |
| total | | 100 |

### [Industrial Applicability]

As described above, the present invention can provide an oil agent for cosmetics that has a good feeling of thickness when applied to the skin in a high temperature and high humidity state, is superior in a feeling of penetration into the skin in a high temperature and high humidity state, and causes less unpleasant sense of adsorption after application. The aforementioned oil agent for cosmetics can be preferably used as an oily starting material of cosmetics, and can be used as a texture improving agent for cosmetics.

In addition, the present invention can provide an emulsion cosmetic that is superior in a feeling of thickness when applied to the skin in a high temperature and high humidity state and a feeling of penetration into the skin in a high temperature and high humidity state, causes less unpleasant sense of adsorption after application, is suitable for use on the wet skin after bathing and the skin in a high temperature and high humidity state after towel drying, and is also superior in the operability of a liquid crystal display screen.

Furthermore, according to the present invention, a powder-containing cosmetic that can suppress a feeling of dryness and a sense of friction due to the inclusion of inorganic powder, and can simultaneously achieve a feeling of thickness and a feeling of blending into the skin when applied to the wet skin can be provided.

The powder-containing cosmetic of the present invention contains an inorganic powder, and can be preferably utilized as sunscreen cosmetics, primer, base makeup cosmetics and the like.

This application is based on patent application No. 2022-086450 and patent application No. 2022-201487 filed in Japan, the contents of which are incorporated in full herein.

## Claims

1. An oil agent for cosmetics, comprising 70% by mass to 99% by mass of (A) a primary alcohol having a branched chain and 20 to 30 carbon atoms, and 1% by mass to 30% by mass of (B) a primary alcohol having a branched chain and 12 to 19 carbon atoms.

2. An emulsion cosmetic comprising 1% by mass to 60% by mass of the oil agent for cosmetics according to claim 1, (C) a divalent to hexavalent polyhydric alcohol having 3 to 6 carbon atoms, and (D) a non-ionic surfactant having an HLB value of 10 to 18, wherein a ratio of the total content of the component (A) and the component (B) to the content of the component (C) [{(A)+(B)}/(C)] is 0.05 to 30 in a mass ratio.

3. A cosmetic comprising 0.5% by mass to 60% by mass of (A) a primary alcohol having a branched chain and 20 to 30 carbon atoms, 0.01% by mass to 30% by mass of (B) a primary alcohol having a branched chain and 12 to 19 carbon atoms, and 2% by mass to 50% by mass of (F) an inorganic powder having an average particle size of 10 nm to 100 µm, wherein a content ratio of the component (A) to the component (B) [(A)/(B)] is 2.3 to 99 in a mass ratio.
